(19) Europäisches Patentamt
European Patent Office
Office européen des brevets

(11) Numéro de publication : **0 487 409 A1**

# (12) DEMANDE DE BREVET EUROPEEN

(21) Numéro de dépôt : **91403117.4**

(22) Date de dépôt : **20.11.91**

(51) Int. Cl.[5] : **C07D 277/30,** C07F 9/54

(30) Priorité : **21.11.90 FR 9014496**

(43) Date de publication de la demande : **27.05.92 Bulletin 92/22**

(84) Etats contractants désignés : **CH DE FR GB IT LI NL**

(71) Demandeur : **ROUSSEL-UCLAF**
**35, Boulevard des Invalides**
**F-75007 Paris (FR)**

(72) Inventeur : **Brayer, Jean-Louis**
**42, rue Jules Dubrulle**
**F-60440 Nanteuil le Haudoin (FR)**
Inventeur : **Demoute, Jean-Pierre**
**65, Avenue Foch**
**F-93360 Neuilly Plaisance (FR)**

(74) Mandataire : **Tonnellier, Marie-José et al**
**111, route de Noisy B.P. no 9**
**F-93230 Romainville (FR)**

(54) **Procédé de préparation de thiazolylalcoxyacrylates et intermédiaires mis en oeuvre.**

(57) L'invention a pour objet un procédé de préparation des composés de formule (I) :

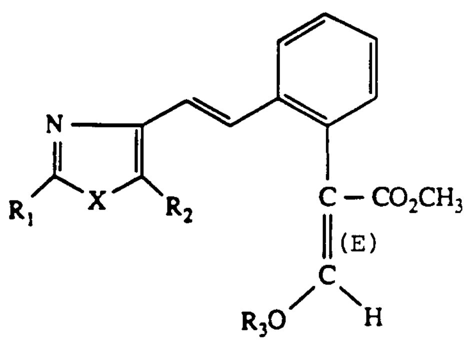

(I)

dans laquelle
— $R_1$ et $R_2$ représentent un atome d'hydrogène, un atome d'halogène, un radical $CF_3$, alkyle, alkényle, alkynyle, alkoxy, thioalkoxy, $SO_2$alkyle, aryle, aryloxy, thioaryle, hétéroaryle, hétéroaryloxy ou hétérocyclique à 5 ou 6 chaînons, éventuellement substitué,
— $R_3$ représente un radical alkyle, alkényle, alkynyle, aryle, alkoxyalkoxyalkyle,
— X représente un atome d'oxygène ou de soufre ou un radical $NR_4$ dans lequel $R_4$ représente un atome d'hydrogène ou un radical alkyle, alkényle, alkynyle, $SO_2$-alkyle, $SO_2$-alkényle, $SO_2$-alkynyle, aryle ou $SO_2$-aryle éventuellement substitué, caractérisé en ce que l'on soumet un halogénure de formule (II) :

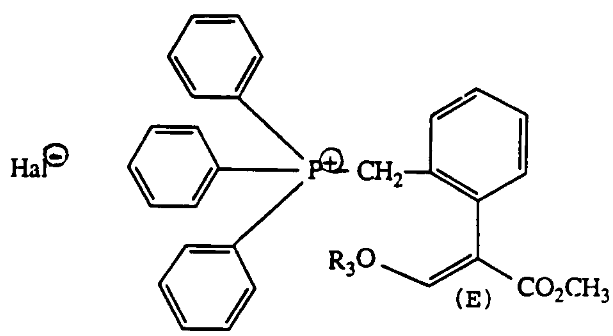

(II)

à l'action d'un composé de formule (III) :

EP 0 487 409 A1

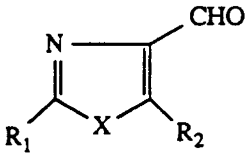

(III)

en présence d'une base, pour obtenir le composé de formule (I) correspondant que l'on sépare si désiré en chacun des isomères.

Les produits de formule sont utiles pour leur activité fongicide.

La présente invention concerne un nouveau procédé de préparation de thiazolylalkoxyacrylates ainsi que les nouveaux produits intermédiaires obtenus.

L'invention a pour objet un nouveau procédé de préparation des composés de formule (I) :

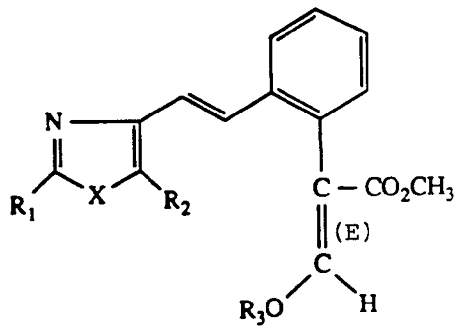

(I)

dans laquelle

– $R_1$ et $R_2$ identiques ou différents représentent un atome d'hydrogène, un atome d'halogène, un radical $CF_3$, un radical alkyle, alkényle, alkynyle ou thioalkyle renfermant jusqu'à 8 atomes de carbone, un radical alkoxy, thioalkoxy ou $SO_2$alkyle renfermant jusqu'à 8 atomes de carbone, un radical aryle, aryloxy ou thioaryle renfermant jusqu'à 18 atomes de carbone éventuellement substitué, un radical hétéroaryle ou hétéroaryloxy éventuellement substitué ou un radical hétérocyclique à 5 ou 6 chaînons éventuellement substitué,

– $R_3$ représente un radical alkyle, alkényle ou alkynyle renfermant jusqu'à 8 atomes de carbone ou un radical aryle renfermant jusqu'à 18 atomes de carbone ou un radical alkoxyalkoxyalkyle renfermant jusqu'à 14 atomes de carbone,

– X représente un atome d'oxygène ou de soufre ou un radical $NR_4$ dans lequel $R_4$ représente un atome d'hydrogène ou un radical alkyle, alkényle ou alkynyle, $SO_2$-alkyle, $SO_2$-alkényle ou $SO_2$-alkynyle renfermant jusqu'à 8 atomes de carbone ou un radical aryle ou $SO_2$-aryle renfermant jusqu'à 18 atomes de carbone éventuellement substitué, la double liaison entre l'hétérocycle à 5 chaînons et le noyau aromatique pouvant être de géométrie E ou Z ou un mélange de géométrie E et Z, caractérisé en ce que l'on soumet un halogénure de formule (II) :

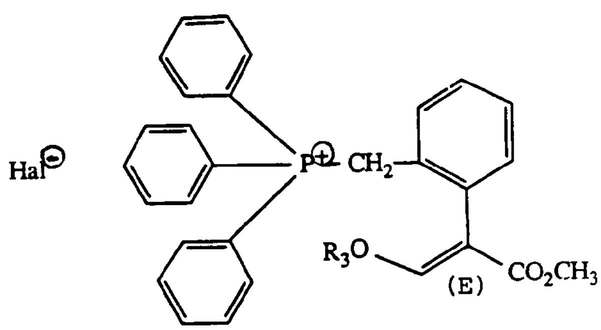

(II)

dans laquelle $R_3$ conserve sa signification précédente et Hal représente un atome d'halogène à l'action d'un composé de formule (III) :

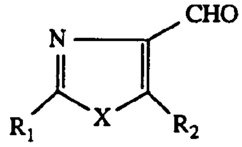

(III)

dans laquelle X, $R_1$ et $R_2$ conservent leur signification précédente, en présence d'une base pour obtenir le produit de formule (I) correspondant sous forme d'un mélange d'isomères que l'on sépare si désiré.

Dans la définition des différents substituants,

– alkyle représente de préférence un radical méthyle, éthyle, n-propyle, isopropyle, n-butyle, isobutyle,

sec-butyle ou tert-butyle,

– alkényle représente de préférence un radical vinyle, allyle ou 1,1-diméthylallyle,

– alkynyle représente de préférence un radical éthynyle ou propynyle,

– alkoxy représente de préférence méthoxy, éthoxy, propyloxy, isopropyloxy, butyloxy linéaire, secondaire ou tertiaire,

– aryle représente de préférence un radical phényle,

– arylalkyle représente de préférence un radical benzyle,

– aryloxy représente de préférence un radical phényloxy,

– hétéroaryle représente de préférence un radical furyle, pyridinyle, pipérazinyle, benzofuranyle, isobenzofuranyle, oxazolyle ou isoxazolyle, thiazolyle, thiophényle,

– hétérocyclique représente de préférence un radical morpholinyle, pyranyle, pyridazinyle,

– alkoxyalkoxyalkyle représente de préférence un radical méthoxyéthoxyalkyle ou tout autre radical de formule :

$$(CH_2)_p\text{-O-}(CH_2)_{p'}\text{-O-alkyle}$$

p et p' identiques ou différents représentant les nombres 1, 2, 3 ou 4 et "alkyle" représentant un radical alkyle renfermant jusqu'à 8 atomes de carbone,

– halogène représente de préférence un atome de fluor ou de chlore.

Dans la définition des substituants alkoxyalkyle, thioalkoxy, $SO_2$-alkyle, $SO_2$-alkényle, $SO_2$-alkynyle, aryloxy, hétéroaryloxy, $SO_2$-aryle, il va de soi que les radicaux alkyle, alkényle, alkynyle, aryle, aryloxy, hétéroaryle représentent de préférence les radicaux cités ci-dessus.

Lorsque les radicaux aryle, aryloxy, thioaryle, hétérocyclique, hétéroaryle, ou hétéroaryloxy sont substitués, ils portent de préférence des substituants choisis dans le groupe constitué par les radicaux hydroxyles libres, estérifiés ou éthérifiés dans lesquels la partie ester ou éther renferme de 1 à 18 atomes de carbone, comme par exemple le radical acétoxy ou le radical méthoxy, les fonctions cétones et oximes, les radicaux alcoyles linéaires, ramifiés ou cyclisés, saturés ou insaturés, comportant jusqu'à 18 atomes de carbone, par exemple le radical méthyle, éthyle, propyle ou isopropyle, le radical éthényle $-CH=CH_2$ ou le radical éthynyle $-C\equiv CH$, les atomes d'halogène, comme le fluor, le chlore, le brome, les groupements $CF_3$, $SCF_3$, $OCF_3$, $NO_2$, $NH_2$ ou $C\equiv N$.

Dans des conditions préférentielles d'exécution du procédé ci-dessus décrit, la base utilisée lors de la réaction des composés (II) et (III) pour préparer les produits de formule (I), peut être par exemple l'hydrure de sodium, un alcoolate alcalin ou alcalino terreux, un amidure alcalin, une amine secondaire ou tertiaire en présence de bromure de lithium.

L'invention a plus particulièrement pour objet :

– un procédé de préparation caractérisé en ce que $R_3$ représente un radical méthyle,

– un procédé de préparation caractérisé en ce que Hal représente un atome de brome,

– un procédé de préparation caractérisé en ce que la base utilisée est une amine secondaire ou tertiaire en présence de bromure de lithium,

– un procédé de préparation caractérisé en ce que la base est l'hydrure de sodium.

L'invention a également pour objet un procédé caractérisé en ce que en outre, on effectue l'inversion de la configuration de l'isomère Z au niveau de la double liaison entre l'hétérocycle à 5 chaînons et le noyau aromatique par action de l'iode.

La partie expérimentale exposée ci-après décrit en détail deux exemples d'une telle isomérisation.

Les produits de formule (I) sont des produits décrits et revendiqués dans la demande de brevet européen publiée sous le n° 0402246.

Ils présentent une activité fongicide intéressante.

Dans la demande de brevet européen citée ci-dessus, est décrit un procédé de préparation des composés de formule (I) telle que définie ci-dessus, selon lequel l'on soumet un composé de formule :

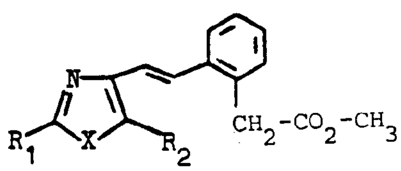

dans laquelle X, $R_1$ et $R_2$ conservent leur signification précédente, à l'action d'une base forte et d'un formiate d'alcoyle ou du N-formylimidazole pour obtenir le composé de formule :

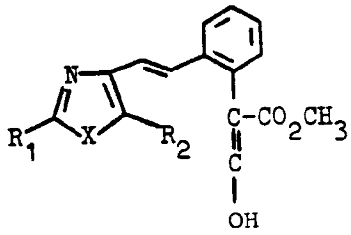

que l'on soumet à l'action d'un agent capable d'introduire le radical $R_3$ puis sépare le cas échéant les différents isomères, et un procédé selon lequel l'on soumet selon la réaction de Wittig-Horner un composé de formule :

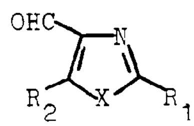

dans laquelle X, $R_1$ et $R_2$ conservent leurs significations précédentes à l'action d'un composé de formule :

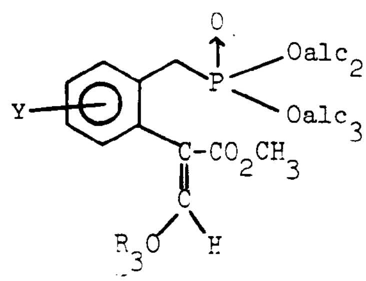

dans laquelle Y et $R_3$ conserve sa signification précédente, $alc_2$ et $alc_3$ identiques ou différents représentent un radical alkyle renfermant jusqu'à 8 atomes de carbone, puis sépare le cas échéant les différents isomères.

Le procédé de la présente invention présente l'avantage d'obtenir les produits de formule (I) avec un très bon rendement.

Les composés de formule (II) :

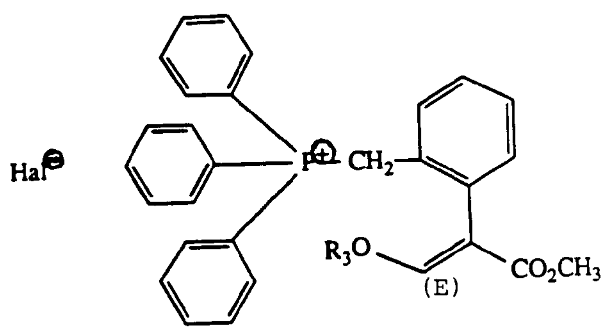

(II)

peuvent être préparés comme indiqué dans la partie expérimentale selon le schéma réactionnel suivant :

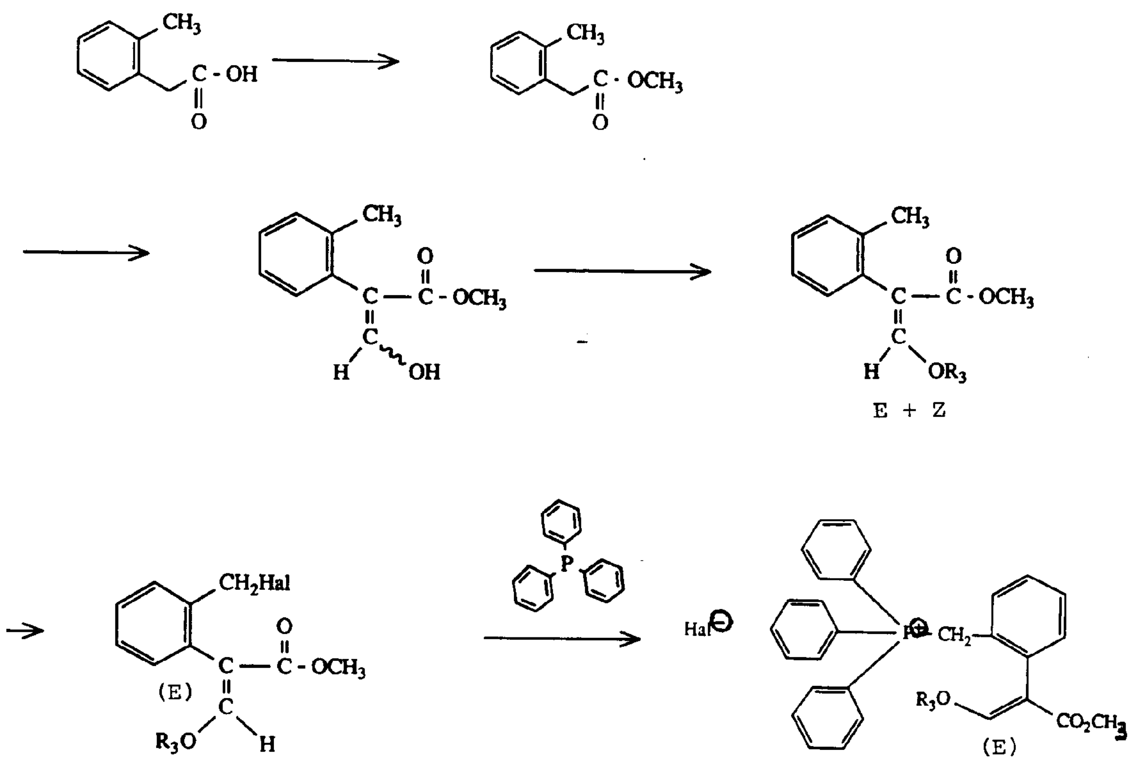

L'invention a donc pour objet les produits de formule (II) telle que définie ci-dessus à titre de produits industriels nouveaux.

L'invention a plus particulièrement pour objet, le composé de formule (II) dont la préparation est donnée ci-après dans la partie expérimentale, à savoir le bromure de [[2-[1-(méthoxycarbonyl) 2-méthoxyéthényl] phényl] méthyl] triphényl phosphonium.

Les composés de formule (III) utilisés au départ du procédé peuvent être préparés comme indiqué dans la partie expérimentale de la demande de brevet européen n° 0402246 citée ci-dessus selon le schéma réactionnel suivant :

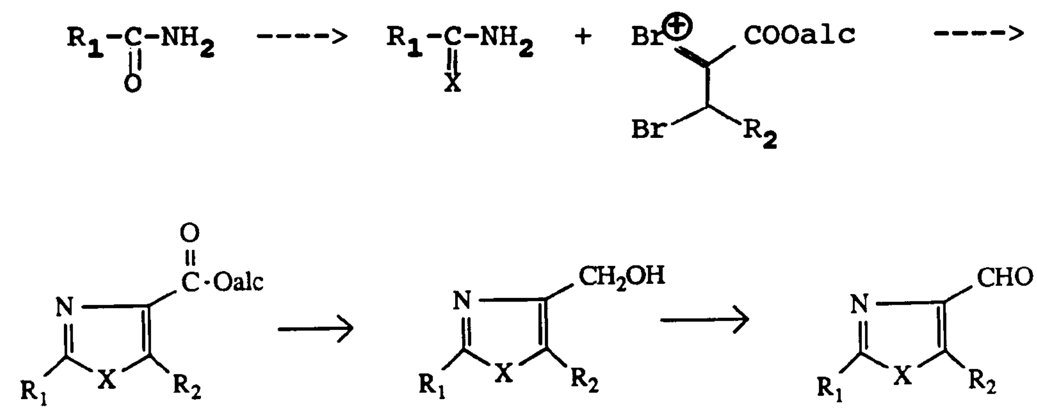

composés de formule (III) dans laquelle $R_1$, $R_2$ et X sont définis comme précédemment.

Les exemples suivants illustrent l'invention sans toutefois la limiter.

**EXEMPLE 1: (E,E) alpha-(méthoxyméthylène) 2-[2-(4-thiazolyl) éthényl] benzène acétate de méthyle**

Mode opératoire 1 :

A un mélange de 10,8 g de bromure de lithium dans 75 cm³ de tétrahydrofuranne, on ajoute à -20°C, 7 ml de diisopropylamine et agite 10 minutes à -10°C.

On verse à 0°C, dans un mélange renfermant 27,5 g de bromure de [[2-[1-(méthoxycarbonyl) 2-méthoxyéthényl] phényl] méthyl] triphényl phosphonium obtenu comme indiqué à la préparation 1, 500 cm³ de tétrahydrofuranne et 5 g de 4-thiazolyl méthanal préparé comme indiqué dans la demande de brevet européen n° 0402246. On maintient le mélange réactionnel sous agitation 2 heures à 0°C puis 48 heures à 20-25°C.

On verse le milieu réactionnel dans l'eau, agite, extrait avec du chlorure de méthylène. On sèche les phases organiques et amène à sec. On obtient 15 g de produit brut que l'on chromatographie sur silice.

On obtient alors 7,5 g d'isomère E,E (F : 117°C) et 2,8 g d'isomère Z,E.

| Constantes RMN | $\delta H_1$ | $\delta H_2$ | $JH_1H_2$ | $\delta H_3$ |
|---|---|---|---|---|
| E,E | 7,10 | 7,42 | 16 | 7,64 |
| Z,E | 6,62 | 6,75 | 12 | 7,49 |

Mode opératoire 2 :

On introduit entre -5 et -10°C, 0,48 g d'hydrure de sodium (à 50 % dans l'huile) dans une solution contenant 5,5 g de bromure de phosphonium (préparation 1) et 1,1 g de 4-thiazolyl méthanal dans 50 cm³ de tétrahydrofuranne et 50 cm³ de diméthylformamide. On maintient le milieu réactionnel 30 minutes à 0°C.

On verse dans l'eau, agite, extrait avec du chlorure de méthylène. On sèche les phases organiques et amène à sec. On obtient 5 g d'une huile brute que l'on chromatographie sur silice comme précédemment, on obtient 1,25 g d'isomère E,E recristallisé dans l'éther isopropylique. F = 117°C, 0,8 g d'isomère Z,E.

Isomérisation de l'isomère Z,E en E,E :

A une solution contenant 0,8 g d'isomère Z,E (préparé précédemment) dans 10 ml de toluène, on ajoute à 20°C, un cristal d'iode. On chauffe 18 heures au reflux puis évapore à sec, on obtient alors une huile brune qui est cristallisée dans l'éther isopropylique.

On récupère 0,53 g de cristaux (F : 119°C) correspondant à l'isomère E,E.

**EXEMPLE 2 : 2-(E) [[[2′-éthyl (2,4′-bithiazol) 4-yl] éthényl] 2-phényl] 3-méthoxy 2-(E) propénoate de méthyle**

A une solution renfermant 6,6 g du produit de la préparation 1), 2,25 g de [2′-éthyl (2,4′-bithiazol) 4-yl] carbaldéhyde (obtenu comme à la préparation 4 de la demande de brevet européen publiée sous le N° 0402246 dans 60 cm³ de tétrahydrofuranne et 60 cm³ de diméthylformamide, ajoute entre -5 et -10°C, 0,6 g d'hydrure de sodium (à 50 % dans l'huile). On maintient 1 heure à 0°C puis verse dans l'eau.

Après extraction à l'éther isopropylique, séchage et concentration, on récupère 4,2 g de cristaux (F = 123°C) que l'on chromatographie sur silice.

On obtient

1,98 g d'isomère E,E (F = 148°C)

0,7 g d'isomère Z,E.

**EXEMPLE 3 : alpha (E) (méthoxyméthylène) 2-(E)-[[2-(2-pentyl) 4-thiazolyl] éthényl] benzène acétate de méthyle**

Mode opératoire 1 :

A un mélange de 6,5 g de bromure de lithium dans 45 cm³ de tétrahydrofuranne, on ajoute à -20°C, 4,2 cm³ de diisopropylamine et maintient en milieu réactionnel 10 minutes, à -20°C.

On verse la solution obtenue, dans un mélange à 0°C, renfermant 16,5 g de produit de la préparation 1, 300 cm³ de tétrahydrofuranne et 5 g de 2-n-pentyl-4-thiazolyl-méthanal, obtenu comme décrit à la préparation

13, de la demande européenne n° 0402246. On agite 2 heures à 0°C, puis 48 heures à température ambiante.

On verse dans l'eau, agite, extrait avec du chlorure de méthylène, sèche les phases organiques et amène à sec. On obtient 20 g de produit brut que l'on chromatographie sur silice.

On obtient alors

5,03 g d'isomère E,E

1,96 g d'isomère Z,E.

| Constantes RMN | $\delta H_1$ | $\delta H_2$ | $JH_1H_2$ | $\delta H_3$ |
|---|---|---|---|---|
| E,E | | | 16 | 7,63 |
| Z,E | 6,54 | 6,65 | 12 | 7,49 |

Mode opératoire 2 :

On ajoute entre -5 et -10°C, 0,48 g d'hydrure de sodium (à 50 % dans l'huile) à une solution contenant 5,7 g de bromure de phosphonium (préparation 1) 1,8 g de 2-n-pentyl-4-thiazolyl-méthanal dans 50 $cm^3$ de tétrahydrofuranne et 50 $cm^3$ de diméthylformamide. On maintient 1 h à 0°C.

On opère comme précédemment et obtient :

1,65 g d'isomère E,E

1,32 g d'isomère Z,E.

Isomérisation de l'isomère Z,E en E,E

A une solution renfermant 1,9 g d'isomère Z,E (obtenu comme ci-dessus), on ajoute un cristal d'iode dans 20 $cm^3$ de toluène. On chauffe aux reflux 20 heures, refroidit et amène à sec. Après chromatographie sur silice, on obtient 1,8 g d'isomère E,E attendu.

**Préparation 1 : bromure de [[2-[1-(méthoxycarbonyl) 2-méthoxy éthényl] phényl] méthyl] triphényl phosphonium**

STADE A : alpha 2-méthyl phényl acétate de méthyle

On porte au reflux pendant une heure un mélange renfermant 136 g d'acide alpha 2-méthyl phényl acétique, 1 400 $cm^3$ de méthanol et 1,4 $cm^3$ d'acide sulfurique concentré. On amène à sec sous pression réduite, reprend avec du chlorure de méthylène, lave à l'eau et sèche la phase organique.

On amène à sec sous pression réduite. On obtient 144 g de produit recherché.

STADE B : 2-(2-méthyl phényl) 3-hydroxy 2-propénoate de méthyle

On introduit 2,9 g d'hydrure de sodium à 50 % dans l'huile dans 60 $cm^3$ de tétrahydrofuranne anhydre. On introduit à 20-25°C, 5 g du produit préparé au stade précédent en solution dans un mélange de 50 $cm^3$ de tétrahydrofuranne et 37 $cm^3$ de formiate de méthyle. On agite le mélange réactionnel pendant 4 heures à 20-25°C et le verse dans 300 $cm^3$ d'acide chlorhydrique 2N. On extrait avec du chlorure de méthylène, sèche la phase organique et amène à sec. On obtient 8,2 g de produit que l'on chromatographie sur silice en éluant avec le mélange hexane-acétate d'éthyle (7-3). On amène à sec et obtient 5,23 g de produit recherché.

STADE C : Z-2-(2-méthyl phényl) 3-méthoxy 2-propènoate de méthyle et isomère E correspondant.

On introduit à 20-25°C dans une solution renfermant 700 $cm^3$ de tétrahydrofuranne et 11 g d'hydrure de sodium, (à 50 % dans l'huile), une solution renfermant 44 g de produit préparé au stade précédent dans 400 $cm^3$ de tétrahydrofuranne. On maintient le mélange réactionnel sous agitation pendant 1 heure et ajoute 150 $cm^3$ d'iodure de méthyle. On agite pendant 5 heures et verse dans l'eau. On extrait au chlorure de méthylène, sèche et amène à sec. On obtient 47,2 g de produit que l'on chromatographie sur silice en éluant par le mélange hexane-acétate d'éthyle (7-3). On obtient :

a) une fraction de rf = 0,3 que l'on amène à sec sous pression réduite. On obtient 1,9 g de produit recherché isomère E.

RMN $CDCl_3$, TMS

H du méthyle du méthylphényle 2,18 ppm
H du phényle 7,07 à 7,22 ppm
H éthylénique 7,55 ppm
H des $OCH_3$ 3,68 et 3,79 ppm

b) une fraction de rf = 0,25 que l'on amène à sec sous pression réduite.
On obtient 36 g de produit recherché isomère Z.

RMN $CDCl_3$, TMS
H du méthyle du méthylphényle 2,22 ppm
H du phényle 7,10 à 7,23 ppm
H éthylénique 6,53 ppm
H des $OCH_3$ 3,70 et 3,91 ppm

STADE D : E-2-(2-bromo méthylphényl) 3-méthoxy 2-propènoate de méthyle.

On porte au reflux pendant 4 heures un mélange de 36 g de produit préparé au stade précédent (delta Z), 500 $cm^3$ de tétrachlorure de carbone, 32,6 g de N-bromosuccinimide et 20 mg d'azodiisobutyronitrile. On refroidit à 20°C, on essore et amène à sec sous pression réduite. On obtient 59 g de produit que l'on chromatographie sur silice en éluant avec le mélange hexane-acétate d'éthyle (7-3). On obtient ainsi 48,5 g de produit (delta E) recherché.

RMN $CDCl_3$, TMS
H de $CH_2Bz$ 4,40 ppm
H du phényle 7,1 à 7,5 ppm
H éthylénique 7,63
H de $OCH_3$ 3,69 et 3,80.

STADE E : Bromure de [[2-[1-(méthoxycarbonyl) 2-méthoxy éthényl] phényl] méthyl] triphényl phosphonium.

On porte au reflux pendant 2 h 30 un mélange de 64 g de produit préparé au stade précédent, 650 $cm^3$ de toluène et 59 g de triphénylphosphine. On laisse revenir le mélange réactionnel à la température ambiante. On essore, lave au toluène et à l'éther isopropylique. On sèche. On obtient 88 g de produit recherché.

Spectre IR
>=O 1700 $cm^{-1}$
C=C 1628 $cm^{-1}$
Aromatique 1600, 1588, 1492, 1482 $cm^{-1}$

RMN - 250 MHz, $CDCl_3$, TMS
H des $CH_3$ 3,44 et 3,90 ppm
H du $-\underline{CH_2}-P^+$ 4,85 ppm
H des phényls 6,85 à 7,8 ppm
H éthylénique 7,49 ppm

**Revendications**

**1)** Procédé de préparation des composés de formule (I) :

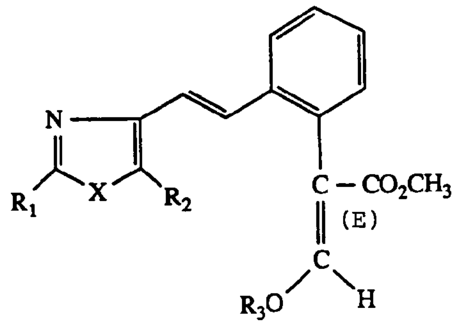

(I)

dans laquelle

– $R_1$ et $R_2$ identiques ou différents représentent un atome d'hydrogène, un atome d'halogène, un radical $CF_3$, un radical alkyle, alkényle ou alkynyle renfermant jusqu'à 8 atomes de carbone, un radical alkoxy, thioalkoxy ou $SO_2$alkyle renfermant jusqu'à 8 atomes de carbone, un radical aryle, aryloxy ou thioaryle renfermant jusqu'à 18 atomes de carbone éventuellement substitué, un radical hétéroaryle ou hétéroaryloxy éventuellement substitué ou un radical hétérocyclique à 5 ou 6 chaînons éventuellement substitué,

– $R_3$ représente un radical alkyle, alkényle ou alkynyle renfermant jusqu'à 8 atomes de carbone ou un radical aryle renfermant jusqu'à 18 atomes de carbone ou un radical alkoxyalkoxyalkyle renfermant jusqu'à 14 atomes de carbone,

– X représente un atome d'oxygène ou de soufre ou un radical $NR_4$ dans lequel $R_4$ représente un atome d'hydrogène ou un radical alkyle, alkényle ou alkynyle, $SO_2$-alkyle, $SO_2$-alkényle ou $SO_2$-alkynyle renfermant jusqu'à 8 atomes de carbone ou un radical aryle ou $SO_2$-aryle renfermant jusqu'à 18 atomes de carbone éventuellement substitué, la double liaison entre l'hétérocycle à 5 chaînons et le noyau aromatique pouvant être de géométrie E ou Z ou un mélange de géométrie E et Z, caractérisé en ce que l'on soumet un halogénure de formule (II) :

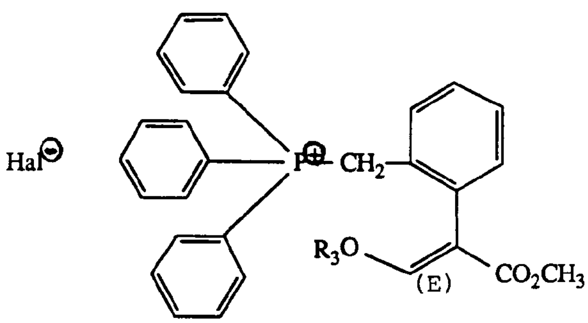

(II)

dans laquelle $R_3$ conserve sa signification précédente et Hal représente un atome d'halogène à l'action d'un composé de formule (III) :

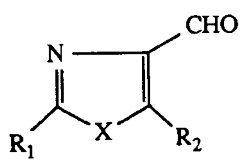

(III)

dans laquelle X, $R_1$ et $R_2$ conservent leur signification précédente, en présence d'une base, pour obtenir le composé de formule (I) correspondant sous forme d'un mélange d'isomères que l'on sépare si désiré.

**2)** Procédé de préparation selon la revendication 1 caractérisé en ce que $R_3$ représente un radical méthyle.

**3)** Procédé de préparation selon la revendication 1 ou 2 caractérisé en ce que Hal représente un atome de brome.

**4)** Procédé selon l'une quelconque des revendications 1 à 3 caractérisé en ce que la base utilisée est une amine secondaire ou tertiaire en présence de bromure de lithium.

**5)** Procédé selon l'une quelconque des revendications 1 à 4 caractérisée en ce que la base utilisée est

l'hydrure de sodium.

**6)** Procédé selon l'une quelconque des revendications 1 à 5, caractérisé en ce que l'on effectue l'inversion de la configuration de l'isomère Z au niveau de la double liaison entre l'hétérocycle aromatique à 5 chaînons et le noyau aromatique par action de l'iode.

**7)** A titre de produits chimiques nouveaux les composés de formule (II) tels que définis à la revendication 1.

**8)** A titre de produits chimiques nouveaux tels que définis à la revendication 7, le bromure [[2-[1-(méthoxycarbonyl) 2-méthoxy éthényl] phényl] méthyl] triphénylphosphonium.

Office européen des brevets

# RAPPORT DE RECHERCHE EUROPEENNE

Numero de la demande

EP 91 40 3117

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| D,P, X | EP-A-0 402 246 (ROUSSEL-UCLAF)<br>* page 6, ligne 33 - page 7, ligne 10; revendication 18 *<br>----- | 1 | C07D277/30<br>C07F9/54 |
| | | | **DOMAINES TECHNIQUES RECHERCHES (Int. Cl.5 )** |
| | | | C07D<br>C07F |

Le présent rapport a été établi pour toutes les revendications

| Lieu de la recherche | Date d'achèvement de la recherche | Examinateur |
|---|---|---|
| LA HAYE | 06 FEVRIER 1992 | HENRY J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)